# FASCICULE DE BREVET EUROPEEN

(11) **EP 0 906 430 B1**
(45) Date de publication et mention de la délivrance du brevet: **26.07.2006**
(21) Numéro de dépôt: 97921933.4
(22) Date de dépôt: 05.05.1997
(51) Int. Cl.: C12N 15/52, C12P 19/42

(54) **PROCEDE DE BIOSYNTHESE PERMETTANT LA PREPARATION DE O-PHOSPHO-L-THREONINE**
BIOSYNTHESE-VERFAHREN ZUR HERSTELLUNG VON O-PHOSPHO-L-THREONINE
BIOSYNTHESIS METHOD ENABLING THE PREPARATION OF O-PHOSPHO-L-THREONINE

(30) Priorité: 13.05.1996 FR 9605896
(43) Date de publication de la demande: 07.04.1999
(73) Titulaire: Aventis Pharma S.A., 92165 Antony Cédex (FR)
(72) Inventeur: BLANCHE, Francis, F-75019 Paris (FR); CAMERON, Béatrice, F-75005 Paris (FR); CROUZET, Joel, F-92330 Sceaux (FR); DEBUSSCHE, Laurent, F-91200 Athis Mons (FR); THIBAUT, Denis, F-75013 Paris (FR); REMY, Elisabeth, F-75005 Paris (FR)
(86) Numéro de dépôt international: PCT/FR1997/000793
(87) Numéro de publication internationale: WO 1997/043421

(56) Documents cités:
- EP-A- 0 462 892
- EP-A- 0 647 717
- WO-A-91/11518
- J. BACTERIOL. (1995), 177(15), 4481-7 CODEN: JOBAAY;ISSN: 0021-9193, XP000616401 POLLICH, MICHAEL ET AL: "Identification and sequence analysis of genes involved in late steps of cobalamin ( vitamin B12 ) synthesis in Rhodobacter capsulatus" cité dans la demande
- J. GEN. APPL. MICROBIOL. (1986), 32(4), 351-9 CODEN: JGAMA9;ISSN: 0022-1260, XP000616396 NOPARATNARAPORN, NAPAVARN ET AL: "Selection of Rhodobacter sphaeroides P47 as a useful source of single cell protein"

## Description

La présente invention concerne un procédé pour améliorer la synthèse in situ de O-phospho-L-thréonine d'un microorganisme producteur de cobalamines.

La vitamine B₁₂ fait partie d'une classe de molécules appelées cobalamines dont la structure est présentée notamment dans WO91/11518.

Les cobalamines sont synthétisées presqu'exclusivement par des bactéries selon un processus complexe également décrit dans WO91/11518. Au niveau industriel, en raison de la grande complexité des mécanismes de biosynthèse, la production de cobalamines et en particulier de la vitamine B₁₂ est principalement réalisée par des cultures en grand volume de bactéries Pseudomonas denitrificans, Propionobacterium shermanii et Propionobacterium freudenreichii.

Il est largement connu que les cobalamines sont synthétisées par certains microorganismes à partir des substrats suivants : l'acide aminolaevulinique, la S-adénosyl-L-méthionine, le cobalt, la glutamine, le R1-amino-2-propanol et le 5,6-diméthylbenzimidazole.

Parmi les précurseurs précédemment cités, le 5,6-diméthylbenzimidazole est synthétisé par les microorganismes producteurs de cobalamines. Deux voies de biosynthèse semblent exister pour le 5,6-diméthylbenzimidazole : l'une est caractéristique des microorganismes aérobies et fait intervenir l'oxygène moléculaire, l'autre est utilisée par les microorganismes anaérobies. Seul un gène intervenant dans la voie anaérobie a été isolé, il s'agit du gène cobT de Salmonella thyphimurium (Trzebiatowski et al., 1994). Aucun gène de synthèse du 5,6-diméthylbenzimidazole n'a à ce jour été identifié chez les microorganismes aérobies. La quantité du 5,6-diméthylbenzimidazole synthétisé par les microorganismes est souvent limitante.

Par conséquent, le 5,6-diméthylbenzimidazole est préparé chimiquement et ajouté dans les milieux de production. La suppression de cet apport dans les milieux présenterait donc un avantage certain.

Jusqu'à présent, aucun procédé de préparation industriel des cobalamines ne mentionne l'addition de précurseurs autres que le cobalt et le 5,6-diméthylbenzimidazole. Certaines souches ne produisant de cobalamines que sur des milieux contenant du R1-amino-2-propanol ont été récemment décrites (Crouzet et al., 1990, Grabau et al., 1992). Le R1-amino-2-propanol pourrait donc être utilisé pour améliorer la production de cobalamines. Cependant, son usage pour une fermentation industrielle éventuelle est délicat et coûteux, car d'une part le R1-amino-2-propanol est un produit irritant et volatile, et d'autre part, il peut inhiber la croissance du microorganisme. Il serait donc particulièrement avantageux de trouver un autre précurseur du résidu R1-amino-2-propanol des cobalamines ne présentant pas ces inconvénients. A cet égard, il est décrit une voie de biosynthèse du R1-amino-2-propanol à partir de la L-thréonine via l'aminoacétone chez certains microorganismes. Mais, la L-thréonine ne permet cependant pas la complémentation des souches citées précédemment.

Plus généralement, pour améliorer la production des cobalamines il peut être avantageux d'augmenter la quantité de leurs précurseurs dans le milieu, en particulier s'ils sont limitants. Cette approche peut être réalisée soit en ajoutant directement le précurseur limitant ou un de ses dérivés ou analogues dans le milieu, soit en amplifiant la synthèse *in situ* de ce précurseur dans la souche productrice en utilisant les techniques de génétique et en particulier la technologie de l'ADN recombinant.

La connaissance des voies de biosynthèse des cobalamines et de leurs précurseurs est à cet effet une étape clé pour améliorer la production des cobalamines.

Ainsi, la plupart des étapes de la voie de biosynthèse de la vitamine B12 ont été récemment caractérisées chez Pseudomonas denitrificans (Blanche et al., 1995). Pas moins de 22 gènes cob impliqués dans la biosynthèse des cobalamines ont été isolés et la fonction de la plupart des polypeptides codés par ces gènes a été identifiée.

D'autres gènes probablement impliqués dans la biosynthèse des cobalamines ou de leurs précurseurs ont été isolés chez d'autres microorganismes. Parfois la fonction de ces gènes n'est pas connue. Seule la détermination précise de leur rôle ou de leur effet permettrait de leur trouver une application. Ainsi chez une bactérie photosynthétique facultative, Rhodobacter capsulatus, un fragment d'ADN portant au moins un gène nécessaire à la formation de l'appareil photosynthétique a été séquencé récemment (Pollich et al., 1993, Pollich et al., 1995a). Il a été suggéré que 5 des 8 gènes isolés sont des gènes de biosynthèse de cobalamines à cause de leur forte homologie avec 5 des 22 gènes cob de P. denitrificans décrits précédemment. Par contre aucune fonction précise n'a pu être directement assignée aux 3 gènes restants, les gènes bluB, bluE et bluF. Les gènes bluB, bluE et bluF incluant leurs séquences promotrices, ont été séquencés et décrits dans Pollich et al., 1995a.

Selon la présente invention, on a découvert un nouveau précurseur de cobalamine. La présente invention a en effet permis d'obtenir une amélioration de production de cobalamine avec des milieux contenant de la O-phospho-L-thréonine. Ce précurseur de cobalamines, non décrit jusqu'à ce jour, a un rôle comparable à celui d'un autre précurseur déjà connu, le R1-amino-2-propanol. Cependant, la O-phospho-L-thréonine possède l'avantage par rapport au R1-amino-2-propanol de ne pas être toxique et d'être un produit facilement manipulable. De plus, son efficacité pour l'amélioration de la production de cobalamines peut être plus de 1000 fois supérieure à celle du R1-amino-2-propanol.

La présente invention a également pour objet l'utilisation d'un fragment d'ADN de Rhodobacter capsulatus pour améliorer ou pour obtenir ou augmenter la synthèse in situ de O-phospho-L-thréonine ou de 5,6-diméthylbenzimidazole d'une cellule donnée.

La présente invention a permis d'obtenir une amélioration de la production de cobalamines avec des milieux ne contenant pas de R1-amino-2-propanol ou de O-phospho-L-thréonine en utilisant un fragment d'ADN portant notamment les gènes bluE et bluF de Rhodobacter capsulatus.

La présente invention a permis enfin d'obtenir une amélioration de la production de cobalamines sur des milieux ne contenant pas de 5,6-diméthylbenzimidazole en introduisant un fragment d'ADN portant notamment le gène bluB de Rhodobacter capsulatus.

La présente invention a pour objet un procédé pour améliorer la synthèse in situ de O-phospho-L-thréonine d'un microorganisme procaryote producteur de cobalamine, caractérisé en ce que on utilise un microorganisme transformé par au moins un fragment d'ADN comprenant les gènes bluE et bluF de Rhodobacter capsulatus et l'on cultive ledit microorganisme dans des conditions permettant l'expression desdits gènes. L'invention a également pour objet un procédé tel que mentionné ci-dessus dans lesquel le microorganisme transformé par au moins un fragment d'ADN comprenant les gènes bluE et bluF de Rhodobacter capsulatus, est en outre transformé par au moins un fragment d'ADN codant pour un enzyme impliqué dans une voie de biosynthèse du 5,6-dimethylbenzimidazole et comprenant le gène bluB de Rhodobacter capsulatus.

Le microorganisme peut contenir de façon endogène un gène tel que caractérisé ci-dessus. Dans ce cas, le procédé selon l'invention permet la surexpression de l'enzyme. Mais ledit microorganisme peut être également exempt de ce type de gène de manière endogène.

Par "fragment d'ADN codant pour un enzyme impliqué dans une voie de biosynthèse de la O-phospho-L-thréonine ou du 5,6 diméthylbenzimidazole", on entend que l'expression dudit fragment d'ADN se traduit par une synthèse de la O-phospho-L-thréonine ou du 5,6-diméthylbenzimidazole dans la cellule, suivie éventuellement d'une libération dans le milieu de culture.

La culture peut se faire en batch ou bien en continu, et la purification des cobalamines peut se faire par les méthodes déjà utilisées au niveau industriel (Florent, 1986).

Dans un mode de réalisation on utilise un microorganisme transformé par un fragment d'ADN codant pour un polypeptide impliqué dans une voie de biosynthèse de la O-phospho-L-thréonine comprenant les gènes bluE et bluF de Rhodobacter capsulatus.

Dans un autre mode de réalisation, on utilise un microorganisme transformé par un fragment d'ADN codant pour un enzyme impliqué dans une voie de biosynthèse du 5,6-diméthylbenzimidazole comprenant le gène bluB de Rhodobacter capsulatus.

L'invention implique l'utilisation d'un fragment d'ADN d'origine naturelle synthétique ou recombinante et homologue. Par fragment, on entend un fragment résultant de la dégénérescence du code génétique

De façon appropriée, on utilise un microorganisme cultivé en aérobiose et ledit fragment d'ADN code pour un enzyme impliqué dans une voie de biosynthèse en aérobiose du 5,6-diméthylbenzimidazole.

La présente invention a également pour objet un procédé de préparation de souches recombinantes de microorganisme procaryote producteur de cobalamine, caractérisé en ce qu'on transforme ledit microorganisme par les techniques de génie génétique par au moins un fragment d'ADN codant pour un enzyme impliqué dans une voie de biosynthèse de la O-phospho-L-thréonine ou du 5,6 diméthylbenzimidazole tel que défini ci-dessus.

La présente invention a également pour objet les souches recombinantes obtenues par le procédé de préparation de souches selon la présente invention.

La présente invention implique l'utilisation dans les procédés selon l'invention d'un ADN recombinant contenant au moins une séquence d'ADN codant pour undit polypeptide et dans lequel la ou lesdites séquences sont placées sous le contrôle de signaux d'expression.

A cet égard, on peut en particulier positionner en 5' de la séquence d'ADN des régions promotrices. De telles régions peuvent être homologues ou hétérologues de la séquence d'ADN. En particulier, des promoteurs bactériens forts, tels que le promoteur de l'opéron tryptophane Ptrp ou de l'opéron lactose Plac de E.coli, le promoteur gauche ou droit du bactériophage lambda, les promoteurs forts de phages de bactéries, telles que les corynébactéries, les promoteurs fonctionnels chez les bactéries gram-négatives, tel que le promoteur Ptac de E.coli, le promoteur PxylS des gènes du catabolisme du xylène du plasmide TOL, le promoteur de l'amylase de Bacillus subtilis Pamy, pourront être utilisés. On peut citer également les promoteurs dérivés de gènes glycolytiques de levure, tels que les promoteurs des gènes codant pour le phosphoglycérate kinase, la glycéraldéhyde - 3 - phosphate déshydrogénase, la lactase ou l'énolase, qui pourront être utilisés lorsque l'ADN recombinant sera introduit dans un hôte eucaryote. Un site de fixation des ribosomes sera également positionné en 5' de la séquence d'ADN et il pourra être homologue ou hétérologue, tel le site de fixation des ribosomes du gène cII du bactériophage lambda.

Des signaux nécessaires à la terminaison de la transcription pourront être placés en 3' de la séquence d'ADN.

L'ADN recombinant utilisé dans les procédés selon la présente invention peut ensuite être introduit directement dans une cellule hôte compatible avec les signaux d'expression choisis ou être cloné sur un vecteur plasmidique pour permettre d'introduire de manière stable la séquence d'ADN en question dans la cellule hôte.

L'invention implique de façon connue l'utilisation des plasmides contenant une séquence d'ADN codant pour undit polypeptide. De façon connue, ces plasmides contiennent aussi un système de réplication fonctionnel et un marqueur de sélection.

Différents types de vecteurs peuvent être utilisés. On préfère dans le cadre de l'invention utiliser des vecteurs de type RK2, c'est-à-dire de vecteurs ayant une origine de réplication RK2. On peut citer à titre d'exemple particulier, le vecteur RK2 (Saurugger et al., 1986), le vecteur pXL435 (Cameron et al., 1989), le vecteur pRK290 (US 4,590,163 ; Ditta et al., 1985) et le vecteur pXL1635 (WO 91/16439). Un vecteur particulièrement avantageux est le vecteur pXL1635. D'autres vecteurs sont décrits dans la demande WO 91/16439.

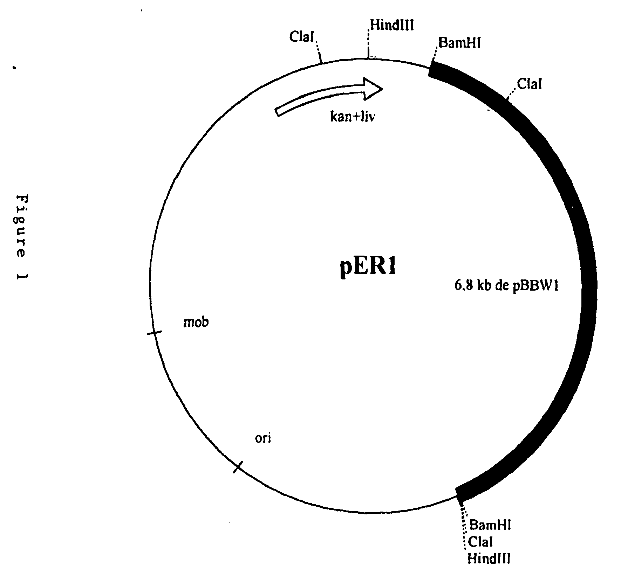

Selon une variante de réalisation, on utilise un microorganisme transformé par un fragment d'ADN comprenant un fragment BamHI de 6,8 kb du plasmide pER1 (figure 1), décrit dans les exemples ci-après et qui code pour la synthèse des deux précurseurs, O-phospho-L-thréonine et 5,6-diméthylbenzimidazole.

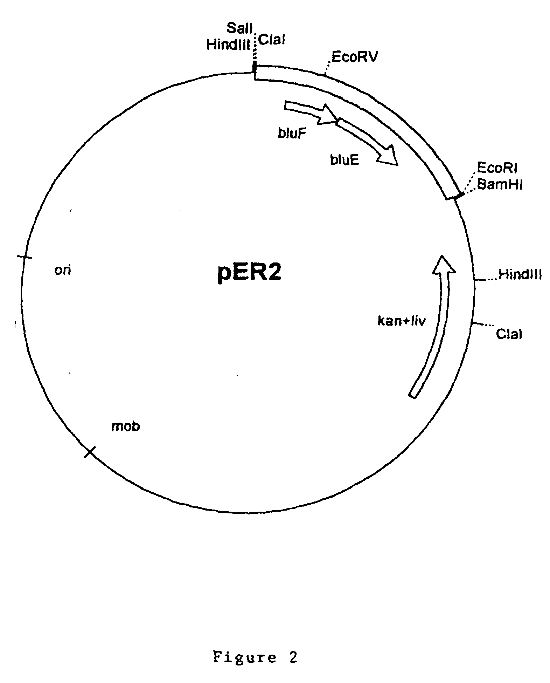

Dans un mode de réalisation plus particulièrement approprié pour l'expression d'un polypeptide impliqué dans la synthèse de la O-phospho-L-thréonine, on utilise un fragment d'ADN comprenant le fragment EcoRI/ClaI de 2.1 kb du plasmide pER2 (figure 2), décrit dans les exemples qui vont suivre.

Dans un mode de réalisation plus particulièrement approprié pour l'expression d'un polypeptide impliqué dans la synthèse de la O-phospho-L-thréonine, on utilise un fragment d'ADN comprenant le fragment EcoRI/EcoRV de 1,6 kb du plasmide pER2 (figure 2) décrit dans les exemples qui vont suivre.

Dans un autre mode de réalisation, plus particulièrement approprié pour l'expression d'un polypeptide impliqué dans la synthèse du 5,6-diméthylbenzimidazole, on utilise un fragment d'ADN comprenant le fragment BamHI de 6,8 kb du plasmide pER1, décrit dans les exemples qui vont suivre.

Les microorganismes procaryotes hôtes qui peuvent être utilisés selon l'invention, sont plus particulièrement les bactéries du genre E.coli, Pseudomonas denitrificans, Agrobacterium radiobacter, Agrobacterium tumefaciens, ou Rhizobium melitoti ou encore Rhodobacter capsulatus. D'autres bactéries sont décrites dans WO91/11518.

Toutefois, avantageusement on utilisera une bactérie P. denitrificans ou A. radiobacter.

D'autres avantages et caractéristiques de la présente invention apparaîtront à la lumière de la description détaillée qui va suivre.

Les exemples 1 et 2 décrivent comment il est possible d'obtenir une production de cobalamines en ajoutant de la O-phospho-L-thréonine dans le milieu de culture d'une souche de Pseudomonas denitrificans ou de Rhodobacter capsulatus. L'exemple 2 montre comment la O-phospho-L-thréonine peut remplacer avantageusement le R1-amino-2-propanol dans les milieux de production, puisque des concentrations au moins 1000 fois plus importanes de R1-amino-2-propanol sont nécessaires pour obtenir une production de cobalamines.

L'exemple 2 montre aussi que la région du chromosome de Rhodobacter capsulatus portant les gènes bluE et bluF est impliquée dans la synthèse de la O-phospho-L-thréonine. L'exemple 3 décrit la construction de plasmides portant les gènes bluE et bluF à partir d'un fragment d'ADN de Rhodobacter capsulatus. Cet exemple 3 montre surtout comment ces plasmides, une fois introduits dans des souches pour lesquelles la production de cobalamines est dépendante de l'addition de R1-amino-2-propanol ou de O-phospho-L-thréonine, permettent d'obtenir une production de cobalamines sur des milieux ne contenant pas de R1-amino-2-propanol ou de O-phospho-L-thréonine. L'exemple 4 montre que la région du chromosome de Rhodobacter capsulatus portant le gène bluB est impliquée dans la synthèse du 5,6 diméthylbenzimidazole.

Liste des figures :
Figure 1 : carte de restriction du plasmide pER1
Figure 2 : carte de restriction du plasmide pER2
Figure 3 : carte de restriction du plasmide pER3

Les figures 1 à 3 représentent les plasmides pER1, pER2 et pER3 respectivement.

### 1. Souches et plasmides

Les souches AH2 et BB1 de Rhodobacter capsulatus (Pollich et al., 1995a) ont été construites à partir de la souche 37b4 (DSM 938). La souche G2650 de Pseudomonas denitrificans a été construite à partir de la souche SBL 27 Rif^{r} par insertion du transposon Tn⁵ (Crouzet et al., 1990). Une souche G2650[Tet] a tout d'abord été construite en utilisant un transposon Tn⁵ portant le gène de résistance à la tétracycline. Puis une souche G2650[Sp] a été construite à partir de la souche G2650[Tet] par échange du gène de résistance à la tétracycline du transposon Tn⁵ par un gène de résistance à la spectinomycine. La souche SBL 27 Rif^{r} dérive de la souche MB 580 (Brevet US, 3 018 225).

Le plasmide pBBW1 a été construit à partir d'un fragment d'ADN de Rhodobacter capsulatus (Pollich et al., 1995a). Le plasmide pAHW25 (Pollich et Klug, 1995a) a été construit à partir du plasmide pBBW1

### 2. Techniques moléculaires

Les techniques générales de manipulation de l'ADN utilisent comme référence le manuel de laboratoire (Sambrook et coll., 1989).

Les enzymes sont utilisés comme le recommande le fabricant et proviennent des laboratoires New England Biolabs et de Boehringer Mannheim.

Les techniques utilisées concernent essentiellement les étapes suivantes :
- digestion par des enzymes de restriction ;
- ligature de molécules d'ADN par la ligase du bactériophage T4.

### 3. Techniques de transformation

La transformation des souches d'E.coli est réalisée par électroporation (Dower et coll., 1988).

### 4. Techniques de conjugaison

Les conjugaisons entre la souche S17-1 d'E.coli et les différentes souches de P. denitrificans sont réalisées selon un protocole adapté de celui décrit par Simon et ses collaborateurs (Simon et coli., 1986). La transformation des souches de *Pseudomonas* peut être réalisée par toute autre technique du génie génétique.

### 5. Préparation des milieux de production de cobalamines

Le milieu utilisé pour la production de cobalamines par les souches de P. denitrificans est le milieu PS4 décrit par Cameron et coll., 1989.

Le milieu utilisé pour la production de cobalamines par les souches de Rhodobacter capsulatus est le milieu RA décrit par Pollich, 1995b.

### 6. Dosage des cobalamines produites

La quantité de cobalamines produite est mesurée soit par dosage microbiologique soit par chromatographie liquide à haute performance (CLHP).

### - Dosage microbiologique :

La quantité de cobalamines produite est mesurée par une méthode semi-quantitative utilisant la souche indicatrice 113-3 d'E.coli, auxotrophe pour la vitamine B12 (Davis et Mingioli, 1950).

Cette souche indicatrice est un mutant metE d'E.coli qui ne possède donc plus qu'une seule homocystéine méthyltransférase (EC 2.1.1.13) qui est B12-dépendante. Sur milieu minimum, elle ne requiert que la présence de vitamine B12 pour pousser. Lorsque cette souche est incluse dans une surcouche de milieu minimum M9 (Miller, 1972) gélosé (auquel il ne manque que la vitamine B12 pour permettre la croissance de la souche), il est possible de doser la vitamine B12. En effet, si l'on dépose à la surface de la surcouche un échantillon d'une solution contenant de la vitamine B12, une auréole de croissance est visible à l'endroit du dépôt après 16 h d'incubation à 37°C. La vitamine B12 contenue dans l'échantillon diffuse et permet la croissance des bactéries incluses dans l'agar. Le diamètre de l'auréole de croissance est proportionnel à la concentration de B12 dans l'échantillon.

Les échantillons sont obtenus par lyse des cellules selon le protocole suivant :

0,1 ml d'une solution de (Tris-HCI pH=8 100 mM, EDTA 20 mM, saccharose 200g/l) contenant 24 mg/ml de lysozyme (Boehringer Mannheim) sont mélangés à 0,5 ml de culture cellulaire à doser. Après 30 min d'incubation à 37°C, 60 µl d'une solution de sodium dodécyl sulfate à 30 g/l sont rajoutés et le mélange est vortexé quelques secondes. 10 µl du lysat cellulaire obtenu, ou éventuellement une dilution au 1/50, sont déposés à la surface de la surcouche.

### - Dosage par CLHP :

Les méthodes utilisées pour le dosage des cobalamines par chromatographie liquide à haute performance sont celles décrites par Blanche et coll.; 1990.

### EXEMPLE 1 : Effet de la O-phospho-L-thréonine sur la production de cobalamines par une souche de Pseudomonas denitrificans.

La souche G2650 [Tet] de Pseudomonas denitrificans est cultivée dans 25 ml de milieu PS4 contenant 2 µg/ml de tétracycline, en Erlenmeyer de 100 ml. Après 24 h de fermentation à 30°C sous agitation (250 rpm), 0,16 ou 0,32 ou 1,5 ml d'une solution de O-phospho-L-thréonine à 10 g/l, ce qui correspond à une concentration finale de 66, 132 et respectivement 600 mg/l, sont éventuellement ajoutés dans le milieu. Les quantités de cobalamines produites dans chacune de ces conditions sont dosées par CLHP au bout de 148 h de fermentation. Les résultats (tableau 1) montrent que la souche G2650 [Tet] ne produit pas de vitamine B12 en milieu PS4. Par contre, la présence de O-phospho-L-thréonine dans le milieu, permet la production de B12 par cette même souche. La quantité de vitamine B12 produite est alors d'autant plus importante que la quantité de O-phospho-L-thréonine rajoutée est importante.

**TABLEAU 1**

| | | | | | |
|---|---|---|---|---|---|
| O-phospho-L-thréonine ajoutée dans le milieu (mg/l) | 0 | 66 | 132 | 600 | 600 |
| | | | | | (ajoutée à t=0) |
| B12 (mg/l) produite | 0 | 1,8 | 2,5 | 4,2 | 3,8 |

### EXEMPLE 2 : Effets comparés de la O-phospho-L-thréonine et du R1-amino-2-propanol sur la production de cobalamines par une souche Rhodobacter capsulatus.

La souche AH2 de Rhodobacter capsulatus est cultivée en Erlenmeyer de 100 ml dans 70 ml de milieu RA en présence de 10 µg/ml de kanamycine et de différentes concentrations de O-phospho-L-thréonine et de R1-amino-2-propanoL Après 24 à 48 h de fermentation à 30°C sous agitation (100 rpm), la quantité de cobalamines produites dans les différentes conditions est mesurée par dosage microbiologique.

Les résultats sont montrés Tableau 2, dans lequel "-" signifie absence de halo de croissance de la souche indicatrice et "+", présence d'un halo de croissance d'un diamètre d'autant plus important qu'il y a de "+". Ces résultats montrent que la souche AH2 de *R. capsulatus,* cultivée en milieu RA ne produit pas de vitamine B12. Par contre, en présence de O-phospho-L-thréonine ou de R1-amino-2-propanol, cette souche est capable de produire de la vitamine B12. Les résultats montrent aussi qu'il faut rajouter de l'ordre 6.10³ fois plus de R1-amino-2-propanol pour avoir le même résultat qu'avec la O-phospho-L-thréonine.

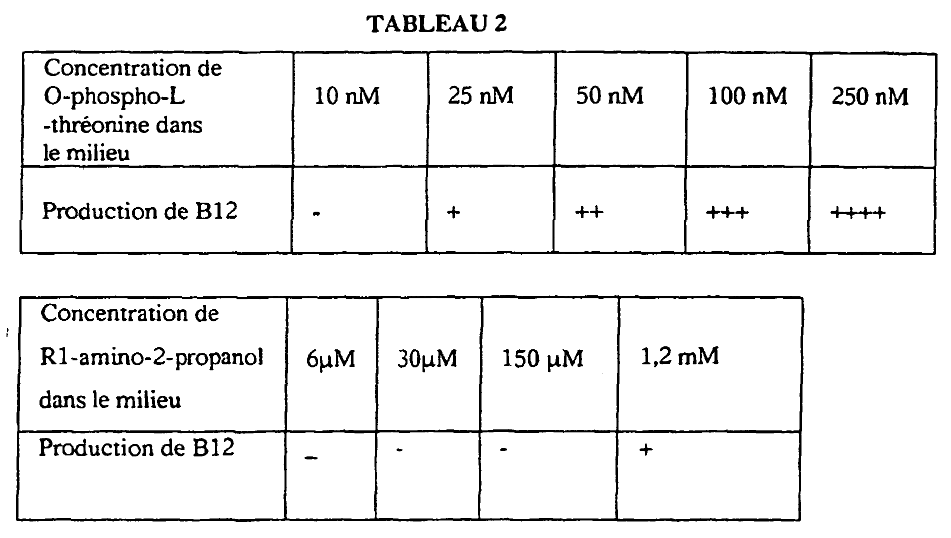

### EXEMPLE 3 : Effet de la présence d'un fragment d'ADN dérivant du plasmide pBBW1 dans la souche G2650, non productrice de cobalamines.

### 3.1. Contruction du plasmide pER1 (figure 1)

Le plasmide pER1 de 17,4 kb a été construit par clonage au site BamHI du vecteur pXL435 (Cameron et coll., 1989) du fragment d'ADN BamHI de 6,8 kb purifié à partir du plasmide pBBW1 (Pollich et Klug, 1995).

### 3.2. Introduction du plasmide pER1 dans la souche G2650 de P. denitrificans

Le plasmide pER1 a dans un premier temps été introduit par électroporation dans la souche S17-1 d'E.coli et, dans un deuxième temps, a été introduit dans la souche G2650 [Tet] de P. denitrificans par conjugaison avec la souche S17-1 d'E. coli contenant le plasmide pER1. Les transconjugants résistant à 50 µg/ml de rifampicine et à 100 µg/ml de lividomycine ont été sélectionnés. 9 clones analysés contenaient le plasmide pER1.

De la même façon, une souche témoin G2650 [Tet] contenant le vecteur pXL435 seul a été construite.

### 3.3. Production de cobalamines par la souche G2650 contenant le plasmide pER1.

Les clones G2650 [Tet] contenant le plasmide pER1 ainsi que 2 clones contenant le plasmide pXL435 ont été cultivés dans le milieu PS4 en présence de rifampicine, tétracycline et de lividomycine. Après 140 h de fermentation à 30°C sous agitation (250 rpm), la quantité de cobalamines produites a été mesurée par dosage microbiologique et par CLHP. Les résultats sont présentés dans le Tableau 3.

**TABLEAU 3**

| | | | |
|---|---|---|---|
| | | B12 dosage microbiologique | B12 CLHP (mg/l) |
| | 1 | ++ | 3 |
| | 2 | ++ | 3,5 |
| | 3 | ++ | 3,4 |
| Souches | 4 | ++ | 3,2 |
| G2650 [Tet] | 5 | ++ | 2,9 |
| contenant le | 6 | ++ | 3,9 |
| plasmide pER1 | 7 | ++ | 3,6 |
| | 8 | ++ | 3,3 |
| | 9 | ++ | 3,9 |
| Souches | 1 | - | O |
| G 2650 [Tet] contenant le | 2 | - | 0 |
| plasmide pXLA35 | | | |
| Souche G2650 [Tet] | | - | 0 |

Alors que ni la souche G2650 [Tet] ni cette souche contenant le plasmide pXL435 ne produisent de vitamine B12 en milieu PS4, cette même souche contenant le plasmide pER1 est capable de produire de la B12 à une concentration de l'ordre de 3,5 mg/l. La souche G2650 [Tet] contenant le plasmide pXL435, c'est-à-dire uniquement le vecteur de clonage qui a servi à la construction du plasmide pER1, ne produit pas de B12 : c'est donc la présence du fragment d'ADN de 6,8 kb dérivant du plasmide pBBW1 qui est responsable de la production de B12.

Ce fragment d'ADN BamHI de 6,8 kb purifié à partir du plasmide pBBW1 confère donc à la souche G2650 [Tet] de P. denitrificans la capacité de produire de la vitamine B12 en milieu PS4.

### 3.4. Sous clonages

Les régions du fragment d'ADN BamHI de 6,8 kb contenant les gènes bluE et bluF ont été sous clonées dans le vecteur de clonage pXL435, donnant naissance aux plasmides appelés pER2 et pER3, grâce à des constructions intermédiaires.

Le plasmide pER2 de 12,9 kb (figure 2) contient le fragment EcoRI/CIaI de 2,1 kb purifié à partir du plasmide pBBW1 et cloné dans le vecteur pXL435. Ce fragment d'ADN a préalablement été cloné aux sites EcoRI/ClaI du plasmide pBluescript II SK+ (Stratagene) puis a été purifié à partir de ce plasmide recombinant sous forme d'un fragment d'ADN BamHI/SalI pour le clonage dans le vecteur pXL435.

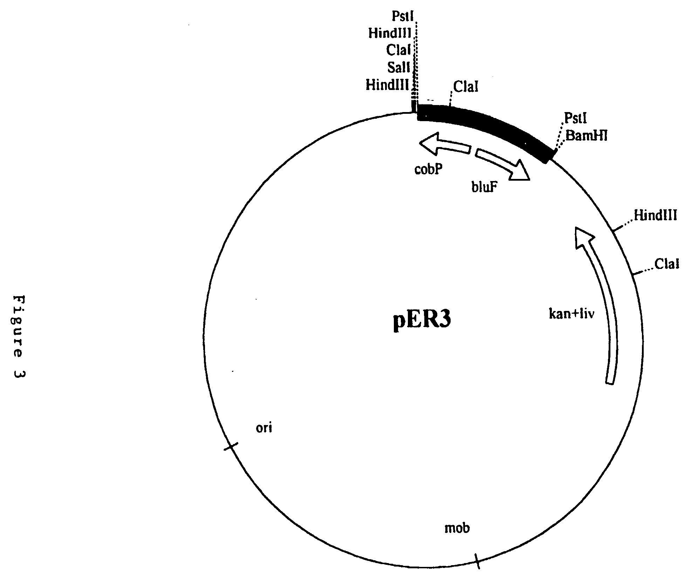

Le plasmide pER3 de 11,9 kb (figure 3) contient le fragment PstI de 1,2 kb purifié à partir du plasmide pBBW1 et cloné dans le vecteur pXL435. Il a été préalablement cloné au site PstI du plasmide pBluescript II SK+ puis a été purifié à partir de ce plasmide recombinant sous forme d'un fragment de restriction BarnHI/SalI pour le clonage dans le vecteur pXL435.

Le plasmide pAHW25 (Pollich et Klug, 1995a) contient le fragment EcoRI/EcoRV de 1,6 kb purifié à partir du plasmide pBBW1 et cloné dans le vecteur pRK415 (Keen et al., 1988) : le gène bluE est alors transcrit à partir du promoteur lac du vecteur.

Les plasmides pER2 ou pER3 ont été introduits dans la souche G2650 [Tet] de P. denitrificans par conjugaison avec la souche S17-1 d'E. coli contenant ces mêmes plasmides. Les clones de G2650 [Tet] contenant les plasmides pER2, pER3 ou pXL435 ont été cultivés dans 5 ml de milieu PS4 en présence de rifampicine, tétracycline et lividomycine. Les plasmides pAHW25 et pRK415 ont été introduits dans la souches G2650[Sp] de P. denitrificans par conjugaison avec la souche S17-1 d'E. coli contenant ces mêmes plasmides. Les clones des souches G2650[Sp] contenant les plasmides pAHW25 ou pRK415 ont été cultivés dans 25 ml de milieu PS4, en présence de rifampicine, lividomycine et spectinomycine. Après 140h de fermentation à 30°C sous agitation (250 rpm), la quantité de cobalamines produites est mesurée par dosage microbiologique et par CLHP.

Les résultats, présentés dans le tableau 4, montrent que seuls les clones des souches G2650 contenant les plasmides pER2 ou pAHW25 sont capables de produire de la vitamine B12 en milieu PS4.

**TABLEAU 4**

| | | B12 dosage microbiologique | B12 en CLHP (mg/l) |
|---|---|---|---|
| Souche G2650[Tet] | | - | 0 |
| Souche G2650[Sp] | | - | 0 |
| Souches G2650[Tet] | 1 | - | 0 |
| contenant le | 2 | - | 0 |
| plasmide pXL435 | 3 | - | 0 |
| | 1 | ++ | 7,4 |
| | 2 | ++ | 5,3 |
| Souches G2650[Tet] | 3 | ++ | 7,9 |
| contenant le | 4 | ++ | 3,5 |
| plasmide pER2 | 5 | ++ | 5,2 |
| | 6 | ++ | 6,8 |
| | 7 | ++ | 7,2 |
| | 1 | - | 0 |
| | 2 | - | 0 |
| | 3 | - | 0 |
| Souches G2650[Tet] | 4 | - | 0 |
| contenant le | 5 | - | 0 |
| plasmide pER3 | 6 | - | 0 |
| | 7 | - | 0 |
| | 8 | - | 0 |
| | 1 | - | 0 |
| Souches G2650[Sp] | 2 | - | 0 |
| contenant le | 3 | - | 0 |
| plasmide pRK415 | 4 | - | 0 |
| | 1 | ++ | 3,0 |
| | 2 | ++ | 2,2 |
| Souches G2650[Sp] | 3 | ++ | 3,0 |
| contenant le | 4 | ++ | 2,9 |
| plasmide pAHW25 | 5 | ++ | 2,9 |
| | 6 | ++ | 3,3 |
| | 7 | ++ | 3,1 |
| | 8 | ++ | 2,4 |

### EXEMPLE 4 : Le gène bluB est impliqué dans la biosynthèse du 5,6-diméthylbenzimidazole (DBI), un précurseur connu de la B12.

La souche BB1 de Rhodobacter capsulatus est une souche mutante qui a été obtenue par insertion d'un interposon dans le gène bluB : c'est une souche bluB⁻ (Pollich et coll., 1995). La souche BB1 est cultivée dans 70 ml de milieu RA en Erlenmeyer de 100 ml en présence de 10 µg/ml de kanamycine et de différentes concentrations de DBI. Les quantités de cobalamines produites par cette souche dans les différentes conditions sont mesurées par dosage microbiologique après 24 à 48 h de fermentation à 30°C sous agitation (100 rpm). Les résultats, résumés dans le Tableau 5, montrent que la souche mutante BB1, non productrice de B12 en milieu RA seul, synthétise cette molécule lorsqu'au moins 14 nM de DBI sont présents dans le milieu. Le gène bluB est donc impliqué dans de la biosynthèse du 5,6-diméthylbenzimidazole.

**TABLEAU 5**

| | | | | | | | |
|---|---|---|---|---|---|---|---|
| concentration de DBI dans le milieu nM | 0 | 7 | 14 | 35 | 70 | 140 | 350 |
| production de B12 | - | - | + | ++ | +++ | ++++ | +++++ |

### REFERENCES

1. Davis B.D. et E. Mingioli (1950), J. Bacteriol. 60 : 17-28
2. Dower W. J., J.F. Miller et C. W. Ragsdale (1988) Nucl. Acids Res. 16 : 6127-6145 Ref M9
3. Sambrook J., E.F. Fritsch et T. Maniatis (1989) Molecular cloning, a laboratory manual, second edition
4. Simon R., M. O'Connell, M. Labes et A. Pühler (1986) Meth. In Enzymology 118 : 640-659
5. Blanche F., D. Thibaut, M. Couder, et J.C. Muller (1990), Anal. Biochem., 189 : 24-29'
6. Blanche F., B. Cameron, J. Crouzet, L. Debussche, D. Thibaut, M. Vuilhorgne, FJ. Leeper, et A. R. Battersby (1995) Angew. Chem. Int. Ed. Engl., 34 : 383-411
7. Cameron B., K. Briggs, S. Pridmore, G. Brefort, et J. Crouzet (1989) J. Bacteriol., 171: 547-557
8. Crouzet J., L. Cauchois, F. Blanche, L. Debussche, D. Thibaut, M.C. Rouyez, S. Rigault. J.F. Mayaux, et B. Cameron (1990) J. Bacteriol., 172 : 5968-5979
9. Grabau C., et J. R. Roth (1992) J. Bacteriol., 174 : 2138-2144
10. Pollich M., S. Jock, et G. Klug (1993) Mol. Microbiol.,10 : 749-757
11. Pollich M., et G. Klug (1995a) J. Bacteriol., 177 : 4481-4487
12. Pollich M. (1995b) Dissertation, Ruprecht-Karls-Universität Heidelberg
13. Florent J., (1986), Vitamins, Biotechnology, vol. 4, VCH Verlagsgesellschaft mbH, Weinheim, In H. -J. Rehm and G. Reed (éd.). 115-158
14. Ditta G., Schmidhauser T., Yakobson E., Lu P., Liang X. W., Finlay D.R., Guiney D., et Helinski D., (1985), Plasmid, 13: 149-154.
15. Miller J. H., (1972), Experiments in Molecular Genetics, Cold Spring Harbor, N.Y.
16. Saurugger P.N., Hrabak O., Schwab H., et Lafferty R.N., (1986). J. Biotechnol. 4 : 333-343
17. Keen N. T., S. Tamaki, D. Kobayashi et D. Trollinger (1988) Gene 70 : 191-197

## Revendications

1. Procédé pour améliorer la synthèse in situ de O-phospho-L-thréonine d'un microorganisme procaryote producteur de cobalamine, **caractérisé en ce que** on utilise un microorganisme transformé par au moins un fragment d'ADN comprenant les gènes bluE et bluF de Rhodobacter capsulatus et l'on cultive ledit microorganisme dans des conditions permettant l'expression desdits gènes.

2. Procédé selon la revendication 1 dans lesquel le microorganisme transformé par au moins un fragment d'ADN comprenant les gènes bluE et bluF de Rhodobacter capsulatus, est en outre transformé par au moins un fragment d'ADN codant pour un enzyme impliqué dans une voie de biosynthèse du 5,6-dimethylbenzimidazole et comprenant le gène bluB de Rhodobacter capsulatus.

3. Procédé selon la revendication 2, **caractérisé en ce que** le fragment d'ADN comprend un fragment BamHI de 6,8 kb du plasmide pER1 représenté figure 1 comportant les gènes bluE et bluF et bluB de Rhodobacter capsulatus.

4. Procédé selon la revendication 1, **caractérisé en ce que** le fragment d'ADN comprenant les gènes bluE et bluF de Rhodobacter capsulatus comprend le fragment EcoRI/CIaI de 2,1 kb du plasmide pER2 représenté figure 2.

5. Procédé selon la revendication 1, **caractérisé en ce que** ledit fragment d'ADN comprenant les gènes bluE et bluF de Rhodobacter capsulatus comprend le fragment EcoRI/EcoRV de 1,6 kb du plasmide pAHW25 ou du plasmide pER2 représenté figure 2.

6. Procédé selon l'une des revendications 1 à 5, **caractérisé en ce que** ledit microorganisme est une souche de Pseudomonas denitrificans ou de Aerobacterium radiobacter.

7. Procédé de préparation de souches recombinantes améliorées pour la synthèse de O-phospho-thréonine **caractérisé en ce qu'**on transforme un microorganisme procaryote producteur de cobalamine par les techniques de génie génétique par au moins un fragment d'ADN comprenant les gènes bluE et bluF de Rhodobacter capsulatus tel que défini dans les revendications 1 à 5.

8. Procédé de préparation de souches recombinantes selon la revendication 7, **caractérisé en ce que** le microorganisme procaryote producteur de cobalamine est en outre transformé par au moins un fragment d'ADN codant pour un enzyme impliqué dans une voie de biosynthèse du 5,6 diméthylbenzimidazole tel que défini dans la revendication 2.

## Claims

1. Method for improving the in situ synthesis of O-phospho-L-threonine of a cobalamin-producing prokaryotic microorganism, **characterized in that** a microorganism transformed with at least one DNA fragment comprising the Rhodobacter capsulatus bluE and bluF genes is used, and said microorganism is cultured under conditions which allow the expression of said genes.

2. Method according to Claim 1, in which the microorganism transformed with at least one DNA fragment comprising the Rhodobacter capsulatus bluE and bluF genes is also transformed with at least one DNA fragment encoding an enzyme involved in a biosynthetic pathway for 5,6-dimethylbenzimidazole and comprising the Rhodobacter capsulatus bluB gene.

3. Method according to Claim 2, **characterized in that** the DNA fragment comprises a 6.8 kb BamHI fragment of the plasmid pER1 represented in Figure 1 containing the Rhodobacter capsulatus bluE and bluF and bluB genes.

4. Method according to Claim 1, **characterized in that** the DNA fragment comprising the Rhodobacter capsulatus bluE and bluF genes comprises the 2.1 kb EcoRI/ClaI fragment of the plasmid pER2 represented in Figure 2.

5. Method according to Claim 1, **characterized in that** said DNA fragment comprising the Rhodobacter capsulatus bluE and bluF genes comprises the 1.6 kb EcoRI/EcoRV fragment of the plasmid pAHW25 or of the plasmid pER2 represented in Figure 2.

6. Method according to one of Claims 1 to 5, **characterized in that** said microorganism is a strain of Pseudomonas denitrificans or of Agrobacterium radiobacter.

7. Method of preparing recombinant strains as are improved with respect to the synthesis of O-phosphothreonine, **characterized in that** a cobalamin-producing prokaryotic microorgansim is transformed, by means of genetic engineering techniques, with at least one DNA fragment comprising the Rhodobacter capsulatus bluE and bluF genes as defined in Claims 1 to 5.

8. Method of preparing recombinant strains according to Claim 7, **characterized in that** the cobalamine-producing prokaryotic microorganism is also transformed with at least one DNA fragment encoding an enzyme involved in a biosynthetic pathway for 5,6-dimethylbenzimidazole as defined in Claim 2.

## Patentansprüche

1. Verfahren zur Verbesserung der *in situ* Synthese von O-Phospho-L-threonin eines prokariotischen Mikroorganismus, der Cobalamin produziert, **dadurch gekennzeichnet, dass** ein Mikroorganismus verwendet wird, der durch mindestens ein DNA-Fragment transformiert wurde, das die Gene bluE und bluF von Rhodobacter capsulatus einschließt, und dass der Mikroorganismus unter Bedingungen kultiviert wird, die die Expression der Gene erlauben.

2. Verfahren nach Anspruch 1, wobei der Mikroorganismus, der durch mindestens ein DNA-Fragment transformiert wurde, das die Gene bluE und bluF von Rhodobacter capsulatus einschließt, ferner durch mindestens ein DNA-Fragment, das ein Enzym kodiert, das an einem Biosyntheseweg von 5,6-Dimethylbenzimidazol beteiligt ist, und das das Gen bluB von Rhodobacter capsulatus einschließt, transformiert wird.

3. Verfahren nach Anspruch 2, **dadurch gekennzeichnet, dass** das DNA-Fragment ein BamHI-Fragment von 6,8 kb des Plasmids pER1 einschließt, das in Figur 1 dargestellt ist, das die Gene bluE und bluF und bluB von Rhodobacter capsulatus einschließt.

4. Verfahren nach Anspruch 1, **dadurch gekennzeichnet, dass** das DNA-Fragment, das die Gene bluE und bluF von Rhodobacter capsulatus einschließt, das Fragment EcoRI/ClaI von 2,1 kb des Plasmids pER2, das in Figur 2 dargestellt ist, einschließt.

5. Verfahren nach Anspruch 1, **dadurch gekennzeichnet, dass** das DNA-Fragment, das die Gene bluE und bluF von Rhodobacter capsulatus einschließt, das Fragment EcoRI/EcoRV von 1,6 kb des Plasmids pAHW25 oder des Plasmids pER2, das in Figur 2 dargestellt ist, einschließt.

6. Verfahren nach einem der Ansprüche 1 bis 5, **dadurch gekennzeichnet, dass** der Mikroorganismus ein Stamm von Pseudomonas denitrificans oder Agrobacterium radiobacter ist.

7. Verfahren zur Herstellung von verbesserten rekombinanten Stämmen zur Synthese von O-Phosphothreonin, **dadurch gekennzeichnet, dass** ein prokariotischer Mikroorganismus, der Cobalamin produziert, gentechnisch durch mindestens ein DNA-Fragment, das die Gene bluE und bluF von Rhodobacter capsulatus einschließt, wie in den Ansprüchen 1 bis 5 definiert, transformiert wird.

8. Verfahren zur Herstellung von rekombinanten Stämmen nach Anspruch 7, **dadurch gekennzeichnet, dass** der prokariotische Mikroorganismus, der Cobalamin produziert, ferner durch mindestens ein DNA-Fragment transformiert wird, das ein Enzym kodiert, das an einem Biosyntheseweg von 5,6-Dimethylbenzimidazol beteiligt ist, wie in Anspruch 2 definiert.
